# EUROPEAN PATENT SPECIFICATION

(11) **EP 0 530 232 B1**
(45) Date of publication and mention of the grant of the patent: **13.12.1995**
(21) Application number: 91909188.4
(22) Date of filing: 30.04.1991
(51) Int. Cl.: B01D 63/02, A61M 1/18, F28F 21/06

(54) **HOLLOW FIBER FLUID TREATMENT APPARATUS AND BLOOD OXYGENATOR**
VORRICHTUNG ZUR FLUIDBEHANDLUNG MIT HOHLFASERN SOWIE VORRICHTUNG ZUR BLUTOXIDATION
APPAREIL DE TRAITEMENT DES FLUIDES AVEC DES FIBRES CREUSES ET OXYGENATEUR DU SANG

(30) Priority: 30.04.1990 US 516960
(43) Date of publication of application: 10.03.1993
(73) Proprietor: BAXTER INTERNATIONAL INC., Deerfield, IL 60015 (US)
(72) Inventor: MATHEWSON, Wilfred, F., Bonsall, CA 92003 (US); RITGER, Philip, L., Huntington Beach, CA 92648 (US)
(74) Representative: MacGregor, Gordon (GB)
(86) International application number: US9103058
(87) International publication number: WO9116967

(56) References cited:
- EP-A- 0 089 122
- EP-A- 0 285 812
- EP-A- 0 345 983
- WO-A-90/04419
- DE-A- 2 839 937
- US-A- 3 557 962

## Description

### BACKGROUND OF THE INVENTION

This invention relates to hollow fiber fluid treatment devices, particularly such devices used for mass or energy exchange through the membrane, i.e. heat exchangers, dialyzers, and in particular membrane oxygenators for the oxygenation of blood in cardiopulmonary bypass blood circuits. Membrane oxygenators direct blood passing through an extracorporeal circuit into contact with a surface or membrane, usually a hollow fiber membrane, through which gas can diffuse or be transferred. The surfaces or membranes are used to transfer oxygen and carbon dioxide between the blood and an oxygen-bearing gas. Devices of this type are disclosed in EP-A-0439528.

Hollow fiber fluid treatment devices have been developed for uses other than oxygenation and some are shown in the following U.S. patents: 3,536,611 to DeFilippi et al., 3,557,962 to Kohl, 3,794,468 to Leonard, 3,957,648 to Roget et al., 4,020,230 to Mahoney, 4,140,637 to Walter, 4,172,794 to Sigdell, and 4,368,124 to Brumfield.

Turning to fluid treatment apparatus for oxygenators, U.S. Patent 4,374,802 to Fukusawa, U.S. Patent No. 4,376,095 to Hasegawa, 4,657,743 to Kanno, and 4,698,207 to Bringham et al. generally disclose an oxygenator with lengthwise-extending hollow fibers which contain the blood; gas is passed over the fibers. U.S. patents 4,620,965 to Fusakawa, 4,659,549 to Hamada, and EPO published application 0,176,651 generally disclose an oxygenator with similar longitudinally extending fibers containing the oxygen-rich gas; blood flows over the fibers. Another disclosure along this line is found in U.S. patent 4,645,645 to Martinez, et al. Yoshida, U.S. Patent 3,998,593 discloses a membrane oxygenator having alternate layers of rectangular polymer membranes and rectangular mesh spacers forming alternate gas channels and blood channels.

Other oxygenators have been developed disclosing helically wound fibers, for example, U.S. Patent 4,306,018 to Kirkpatrick, U.S. Patent 4,715,953 to Leonard, JP-A-63139562 to Japan Medical Supply Co., Ltd., U.S. Patent 4,424,190 to Mather III, Bringham et al., mentioned above, and U.S. patent 4,808,378 to Nakanishi. An invention directed to a method of winding a bundle of hollow fibers around a core for an oxygenator is disclosed in U.S. patent 4,572,446 to Leonard.

Blood is passed through a tubular membrane containing a group of capillary membranes extending spirally through it in U.S. patent 3,893,926, to Awad, and a somewhat similar arrangement is said to be applicable to oxygenators in U.S. patents 3,963,622 and 4,246,120 to Baudet, et al.

A diffusion membrane unit said to be applicable to oxygenators is disclosed in U.S. patent 4,346,006 to Kopp, et al. The unit includes a plurality of separate, parallel membrane tubes formed into a flat, substantially two-dimensional array joined by adhesive in spaced relation to each other for improved exposure of the exterior of the tubular membrane to the passing fluid. The adhesive may be a material similar to the membrane material and extends continuously across the parallel membrane tubes. In another embodiment, the unit contains a plurality of planar, single-layered arrays of parallel capillary tubes defining out of phase sinuous paths.

A device for the extracorporeal treatment of blood is disclosed in U.S. patent 4,428,403 to Lee, et al. It consists of hollow tubing with plastic monofilament wrapped around its outer surface in a generally spiral configuration, preferably then formed into a coil in which the coils are spaced apart by the monofilament.

A membrane oxygenator is also disclosed in U.S. patent 4,622,206 to Torgeson. It has a housing with hollow fibers extending cross-wise, where the blood flows transversely over the fibers. The fibers are placed in a layer at a center to center spacing of about 1.5 to 4 times the diameter of the fibers. Filaments may be positioned between at least some of the fibers to improve spacing and facilitate more efficient transfer. Fibers may be wound around a frame member and adjacent layers preferably have fibers angularly offset with respect to each other.

One primary disadvantage, however, of commercially available membrane oxygenators containing membrane units such as the above pertains to the relatively large "priming volume" of the extracorporeal circuit which includes such devices. The total internal volume of the extracorporeal circuit, which includes the oxygenation and heat exchange devices as well as other devices, is flushed out before surgery to remove any extraneous gas from the extracorporeal circuit. "Priming" is typically performed with a biocompatible solution, e.g. a saline solution, which then mixes with the patient's blood, causing dilution of the blood cells, and in particular the red blood cells for a given volume of fluid. To minimize such hemodilution, donor blood may have to be introduced into the diluted blood passing through the extracorporeal circuit.

While the addition of donor blood may reduce hemodilution, it presents other complications, such as compatibility problems between the donor blood and patient's blood and complications associated with blood-borne diseases. As a result, other measures can be taken to correct the effects of hemodilution, such as using hemoconcentrators to concentrate the blood cell count. However, they are expensive and cumbersome to operate, and may injure the blood cells.

It is thus apparent that it would be advantageous to design a very efficient hollow fiber membrane apparatus in order to reduce the priming volume of the oxygenator. The major impediment to doing so, however, is the requirement of providing sufficient surface area for both heat exchange and for oxygenation. As to oxygenation, this requires a well-designed hollow fiber fluid treatment apparatus within the oxygenator to obtain sufficient oxygen transfer rates.

Another problem which may occur with membrane oxygenators is injury to or deterioration of the blood as it passes through or over the hollow fiber membranes. Stagnant areas result in thrombogenicity, while rapid flow rates in some areas result in shear stresses which cause hemolysis. Thus, it is desirable to produce a hollow fiber system with as uniform and even blood flow as possible, in order to minimize blood injury.

### SUMMARY OF THE INVENTION

The present invention overcomes the above-mentioned problems and disadvantages of hollow fiber fluid treatment devices and membrane oxygenators which utilize them. In the present invention, in one aspect, the membrane arrangement is designed to provide a very high membrane surface area for fluid contact in a minimum volume, with thin, uniform fluid flow throughout.

For the purpose of the present invention, the term "gas permeable membrane", "hollow fiber", or "hollow fiber membrane" is meant to include any substrate formed from the material which functions to allow for the transfer or diffusion of gas from one side of the substrate to the other and, in the particular application, a substrate formed from a material which allows for transfer or diffusion of oxygen and carbon dioxide gas.

DE-A-2839937 discloses a hollow fibre dialyser in which parallel, enclosed dialysate channels are provided by partition ribs or spacer fibres and sheets engaging the ribs or spacer fibres. Each channel contains a membrane fibre. Where spacer fibres are provided, alternating with the membrane fibres, they are of larger diameter than the membrane fibres to define the channels.

The precharacterising part of Claim 1 is based on this disclosure and the characterising features of Claim 1 define the distinguishing features of the invention.

The invention provides fluid treatment apparatus including a hollow fiber membrane device for the transfer of mass or energy through the device and comprising a housing having first fluid inlet and outlet means and second fluid inlet and outlet means, the device comprising hollow fiber membranes extending substantially longitudinally within said housing; said first fluid inlet and outlet means being connected with the hollow fiber membranes for passage of a first fluid therethrough and said second fluid inlet and outlet means being arranged to pass a second fluid over the exterior of the membranes for mass or energy transfer through the membrane, first inert fibres located between said hollow fiber membranes, extending substantially longitudinally and comprising a warp with said hollow fiber membranes; and second inert fibers extending generally transverse to the hollow fiber membranes, generally contiguous therewith and comprising a weft to form a weave with the hollow fiber membranes and first inert fibers.

Usually the hollow fibers extend through the weave aligned straight relative to the first inert fibers, which follow an oscillating path about the second inert fibers. The first inert fibers are spaced so that the warp consists of alternate strands of hollow fiber and first inert fiber and the size of the first inert fibers defines (at least in part) the size of the fluid film to be passed over the hollow fiber membrane for mass or energy transfer through the membrane. For use in an oxygenator, biocompatible materials may be used, preferably microporous polypropylene for the hollow fibers and monofilament polymers for the inert fibers because the spacing between the hollow fibers can thus be closely controlled. Most preferably, the monofilaments are polyester.

The invention also resides in a membrane oxygenator containing such a hollow fiber weave for oxygenation of (and removal of carbon dioxide from) the blood. In this aspect, the oxygenator consists of apparatus having a blood inlet and outlet and a gas inlet and outlet and also having at least one channel with a base which forms a primary conduit for a second fluid, where the channel contains the hollow fiber weave described above with the hollow fibers extending substantially lengthwise through the channel to form a conduit, preferably for the oxygen-containing gas.

Preferably, the first inert fibers are each about one fifth to one third the diameter of the hollow fibers and the second inert fibers are each about one eighth to one fifth the diameter of the hollow fibers, and spaced about five to the inch to promote the flow of thin films of blood and high oxygen transfer rates. Generally, the hollow fibers are disposed in the channel in woven ribbons sized to fit the channel and arranged in alternating layers, the first inert fibers in alternate layers being about one third the diameter of the hollow fibers, and the first inert fibers in the remaining layers being about one fifth the diameter of the hollow fibers so that the hollow fibers in adjacent layers are generally offset from each other to promote even spacing between the layers and even blood flow throughout. In other aspects, the first inert fibers alternate in size in each ribbon so that the ribbons can be layered with the hollow fiber membranes of each layer offset from those of adjacent layers.

In the preferred membrane oxygenator, the woven ribbons are disposed in the channel spaced from the base with a spacer to define a gap adjacent the base. The gap forms a first channel for blood passage, where the bulk of the heat exchange occurs, the second channel for blood passage, directed primarily to oxygenation, being through the ribbons. Preferably, the hollow fibers are disposed in the channel in about thirty-two woven ribbons having either about 6 or about 7 hollow fibers each, and a coiled spacer extends the length of the channel in the gap to maintain the integrity of the gap and the position of the ribbons during manufacture and use of the oxygenator. In a preferred aspect, the invention includes a blood oxygenator and heat exchange device containing a housing defining a first internal chamber, and a first thermally conductive core positioned inside the housing internal chamber, the core being formed with an internal region through which a heat exchange fluid can be directed and a first external surface with a plurality of separate, adjacently positioned parallel blood receiving channels. A hollow fiber membrane fluid treatment apparatus described above is is arranged in each of the channels, each hollow fiber membrane defining a separate gas flow pathway which is open at opposite ends of said membranes. A gas manifold associated with the housing has two gas channels, each gas channel communicating with ends of the hollow fiber membranes and ports through which gas can be introduced or removed. A blood manifold means is mounted in the housing and adjacent to the core for delivering blood to the blood receiving channels.

### DESCRIPTION OF THE DRAWINGS

The present invention may be better understood and the advantages will become apparent to those skilled in the art by reference to the accompanying drawings, wherein like reference numerals refer to like elements in the several figures, and wherein:
FIGURE 1 is a perspective side view of a blood oxygenator/heat exchange device in accordance with an embodiment of the invention;
FIGURE 2 is a cross-sectional view of FIGURE 1 along line 2-2;
FIGURE 2A is an enlarged view of the blood receiving channels, as defined by the pleats of the bellows and the hollow fiber membranes, as seen generally in FIGURE 2;
FIGURE 3 is an exploded view of the device of FIGURE 1;
FIGURE 4 is a cross-sectional view of FIGURE 1 along line 4-4;
FIGURE 5 is a side elevation of the preferred hollow fiber weave of the present invention.
FIGURE 6 is a cross-section of the hollow fiber weave of the present invention taken at lines 10-10 of Figure 5.
FIGURE 7A is a cross-section of a preferred hollow fiber fluid treatment apparatus of the present invention layered in a blood flow channel of an oxygenator.
FIGURE 7B is a schematic cross-section of three layers of ribbon of the preferred fluid treatment apparatus showing the relative spacing of the inert fibers and the hollow fibers which are layered in the blood flow channel.
FIGURE 7C is a schematic cross-section of a ribbon of the present invention showing an alternate spacing arrangement.
FIGURE 8 is a side elevation of the preferred heat exchange jacket for the present oxygenator.
FIGURE 9 is a side elevation of the preferred blood manifold of the oxygenator of the present invention.

### DESCRIPTION OF THE PREFERRED EMBODIMENTS

The present invention is directed to a hollow fiber membrane fluid treatment apparatus for the transfer of material (or energy in the case of a heat exchange device) between one fluid and another. In the preferred embodiment, it is a hollow fiber weave for the oxygenation of blood in a membrane oxygenator, and an oxygenator containing such a hollow fiber fluid treatment apparatus.

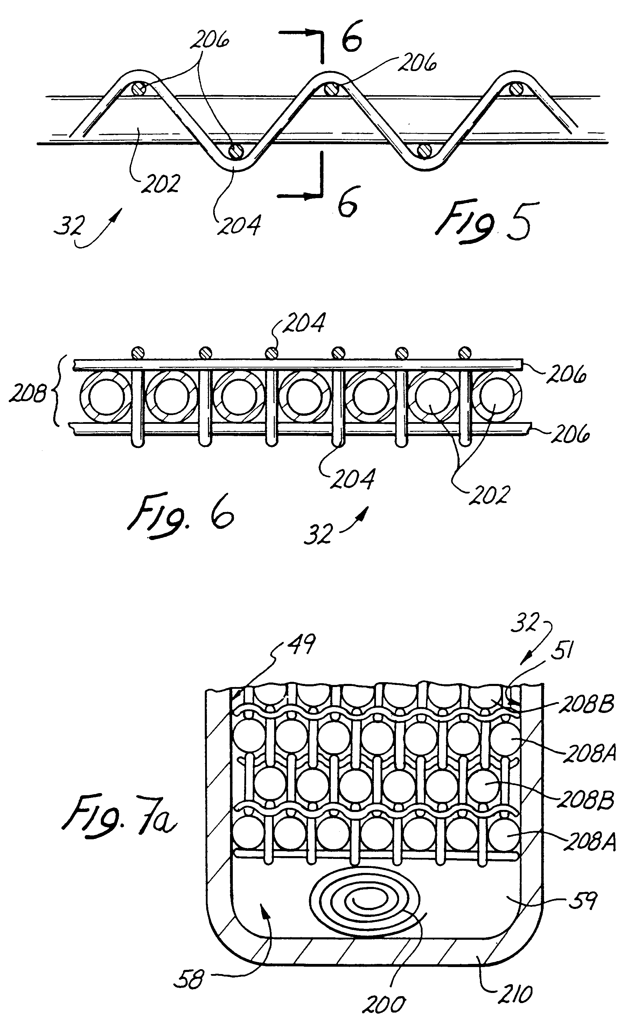

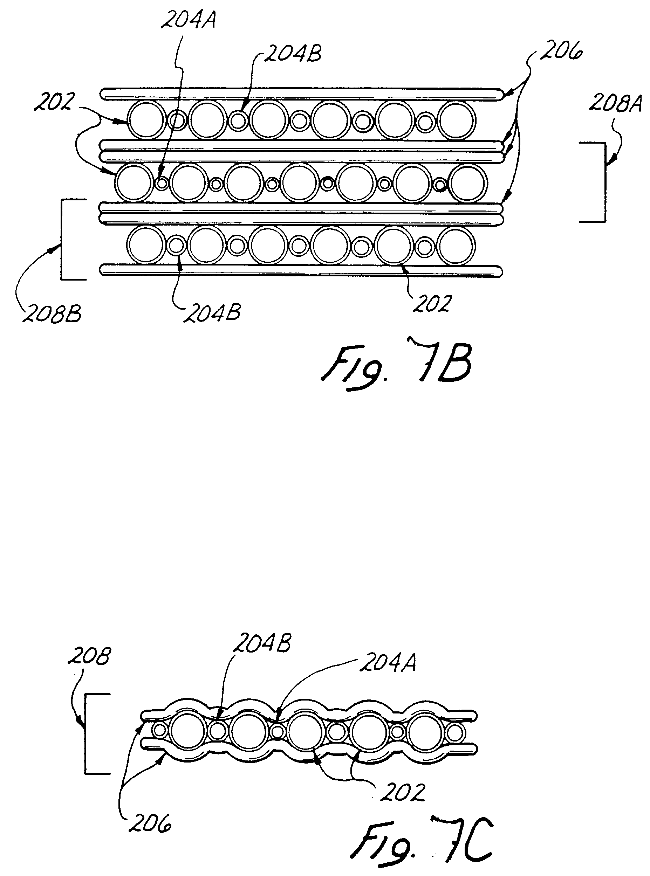

Referring first to Figures 5 and 6, the preferred hollow fiber fluid treatment apparatus can be seen. The fluid treatment device includes a bundle 32 of hollow fiber membranes 202. The hollow fibers 202 extend generally parallel to each other in a substantially longitudinal direction and are spaced between first inert fibers 204, preferably monofilaments, also extending substantially longitudinally. In the preferred embodiment, fibers 202 and 204 form the warp of what amounts to a hollow fiber weave or, most preferably, woven ribbon 208. Second inert fibers 206 are also included; they usually extend transverse and contiguous to the hollow fibers 202, preferably spaced alternately above and below fibers 202 to form the weft of weave or ribbon 208. The first inert fibers 204, preferably flexible relative to the hollow fibers 202, pass above and below the second inert, or weft, fibers 206 in a sinuous or oscillating path.

In the preferred fluid treatment apparatus of the present invention, the hollow fibers form a conduit for oxygen gas, oxygen-rich gas, or oxygen-containing gas, (all hereafter referred to as "oxygen-containing gas") to oxygenate the blood, which passes over the surface of the fibers. In addition, carbon dioxide is removed from the blood through the membrane. Useful membrane materials for the hollow fiber membranes may be silicone, polyolefins such as polypropylene or polyethylene or another suitable hydrophobic polymeric material. These types of membranes will allow oxygen to pass from an oxygen-bearing gas to the blood, which is oxygen deficient, while allowing for the transfer of carbon dioxide from the blood to the gas. In general, the porosity of the membrane is selected according to the requirements of the invention, and in the present case, the membrane selected should be biocompatible and allow for the selective diffusion of oxygen and carbon dioxide while inhibiting the passage of liquids.

The types of hollow fiber membranes useful for the practice of the invention are well known, with such membranes described in any one of the above mentioned US-A-4715953, US-A-4376095, US-A-4657743 and US-A-4424190. All such relevant disclosures to such membranes are incorporated herein by reference. As is well known, however, the rate of gas exchange is dependent upon the permeability of the membrane, as well as the partial pressure of the gases being exchanged within the associated fluid, that is, the oxygen-bearing gas and the blood. For a more detailed description of such membranes, and theory of operation, see "Cardiopulmonary Bypass", 2nd Edition, Charles C Reed and Trudi B. Stafford, Texas Medical Press, Inc, 1985, Chapter 28 "Membrane Oxygenator", pages 427-449.

The preferred material for the hollow fiber membrane of the present invention is microporous polypropylene, in particular Oxyphan membrane, Oxyphan being a registered trademark of ENKA AG. Oxyphan is a hydrophobic microporous polypropylene membrane made by a thermally induced phase separation process. The preferred form of Oxyphan is Oxyphan 50/280, meaning that the internal diameter of the hollow fibers is about 280 microns while the wall thickness is about 50 microns. However, for a blood oxygen transfer application such as the present one, the internal diameter range for the hollow fibers will generally be about 100-500 microns, preferably 200-400 microns and optimally about 280 microns, while the wall thickness range will be about 30-80 microns, preferably about 35-60 microns and most preferably about 50 microns. In the case of the wall thickness, a trade off is made between gas pressure within the fiber and stability of the structure; below about 30 microns in wall thickness, the hollow fibers may become too fragile for easily handling. The void volume is preferably about fifty (50) percent.

Turning to the first inert fibers 204 (which form part of the warp of ribbon 208), they are preferably flexible relative to the hollow fiber membrane itself and formed of a biocompatible material such as polyester, nylon or polypropylene; the preferred polymer is polyester and the preferred first inert fibers 204 are made of polyester monofilament. Different materials, of course, can be used for the first inert fibers 204 but, in general, these materials, if used in an oxygenator, must be biocompatible, strong, and easy to handle. The use of monofilament for the first inert fibers is very useful in an oxygenator such as the present one because it can be obtained at very close tolerances, and can be used to define a ribbon 208 with very precise spacing between the hollow fibers, to optimize the uniformity of the film of blood flowing over the hollow fibers for optimum gas exchange and minimum blood injury.

In the present case, first inert fibers 204 are preferably about 1/10 to 1/2 the diameter of the hollow fiber membrane, more preferably 1/5 to 1/3 the diameter of the hollow fiber membrane 202. This precise sizing of monofilament 204, plus the generally sinusoidal or oscillating arrangement of the monofilament 204 relative to the hollow fiber membrane 202, allows passage of the blood through the weave 208 immediately adjacent to and over the hollow membranes 202 in extremely thin films defined at least in part by the size of the fibers 204. Blood films so thin are unusual and provide very effective oxygenation. In the preferred embodiment, the first inert fibers in each ribbon 208 are either one-fifth or one-third the diameter of the hollow fibers; in alternate embodiments, they may alternate within a single ribbon 208 to vary the spacing between the hollow fibers.

The second inert fibers 206, the weft fibers, can be formed of the same materials and in the preferred embodiment are similarly formed of polyester monofilament. They stabilize the weave and position the first inert monofilament fibers 204 in their oscillating path above and below the plane of the hollow fibers. In general, the membrane fiber 202 is much larger in width than the second inert fibers 206. The weft fibers 206 are positioned preferably every 1.25 to 0.15 cm (1/2 to 1/16 inch) along the length of the membranes, most preferably about 0.5 cm (1/5 inch) apart, and preferably have a diameter 1/20th to 1/5th, most preferably 1/8th, that of the hollow fiber 202.

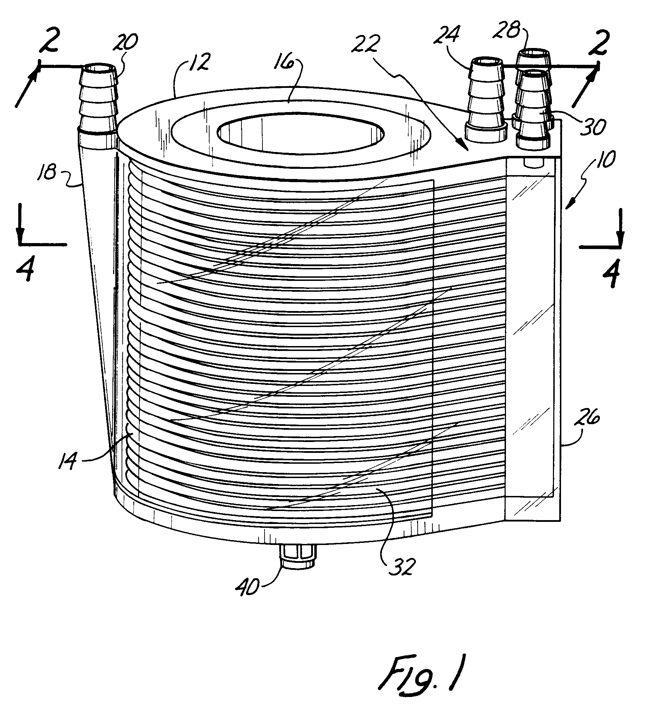

Turning now to Figure 1, a specific embodiment of the heat exchanger/oxygenator containing the present fluid treatment apparatus will now be described. The heat exchanger/oxygenator is seen generally at 10. It will be connected in an extracorporeal circuit for the purpose of raising or lowering the blood temperature, and performing the necessary gas exchange. The device 10 generally includes an outer housing enclosure 12, in which is mounted a heat exchange body, or bellows 14 and a heat exchange jacket 16. An alternate preferred heat exchange jacket 216 is shown in Figure 8.

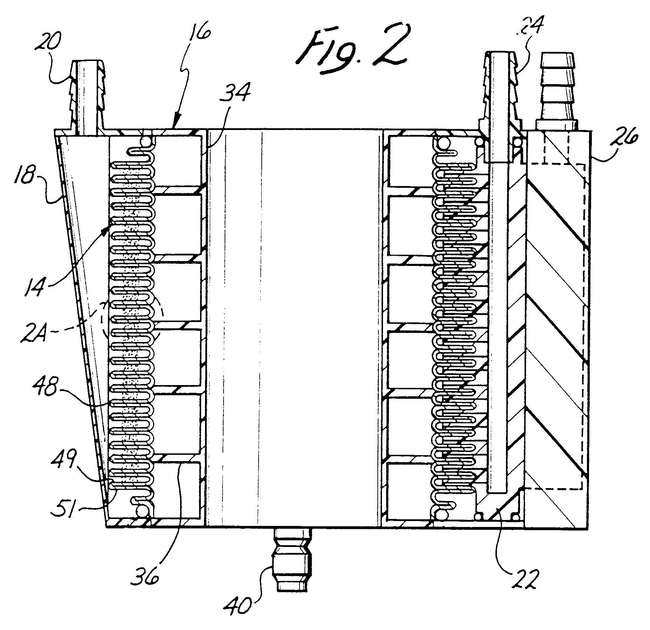

The bellows 14 is formed with a plurality of pleats 48, the precise number of which is dependent on the desired characteristics of the oxygenator with a blood receiving channel defined between each of the adjacently positioned pleats 48, such channels best seen in Figure 7a and 2A at 58. The illustrated blood receiving channels 58 are arranged about the circumference of the cylindrically shaped bellows 14 in a substantially parallel orientation. Blood is directed through each of the channels 58 for the purpose of performing the heat exchange and gas exchange.

Each pleat 48 is defined by two walls, seen generally at 49 and 51, which are usually angularly positioned with respect to each other. The inner surface of the walls 49 and 51 define a space 50, which is positioned along the inner side of the bellows 14 opposite channels 58. Space 50 faces jacket 16 to define a portion of a fluid pathway. Fluid travels through this space 50 and comes into intimate contact with walls 49 and 51. The opposite surfaces of these walls 49 and 51 define the blood receiving channels 58. The blood passing through the blood receiving channels 58 will thus come into intimate contact with the other surfaces of the walls 49 and 51 to ensure efficient cooling or heating of the blood.

The heat exchange jacket 16 or 216, which is positioned within the bellows 14, defines a fluid passage, through which a heat exchange fluid passes to heat or cool the bellows 14. The heat exchange jacket 16 is designed to direct the fluid against the inside surface of the heat exchange body 14, which is formed from a thermally conductive material, i.e. metal. The fluid will thus either heat or cool the body 14, with the blood being directed across the surface of the heat exchange body 14 to effect the necessary heat exchange. Heat exchange jacket 16 directs the fluid from entry 40 through a pathway 37 defined by lips 36 extending from a cylindrical wall 34, to an outlet 46, while heat exchange jacket 216 directs the fluid over its smooth surface from entry 240 and the bellows (to be discussed) channelling the fluid and forming a fluid path to outlet 246.

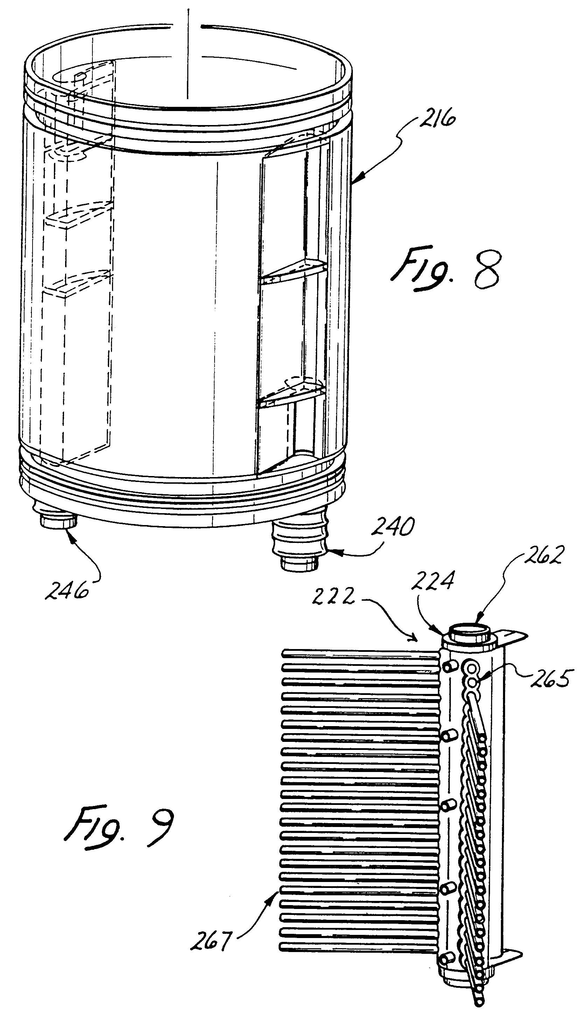

The housing enclosure 12 is also formed with a blood exit manifold 18, and exit tubing connector 20. Blood passing through the device 10 will collect in the exit manifold 18 and exit through the tubing connector 20. A tube, not shown, coupled to the tubing connector 20, will direct the blood back to the patient, or to another device in the extracorporeal circuit, not shown. Blood enters the device 10 through a blood manifold, seen generally at 22 (or 222 in Figure 9) which is fitted into the housing enclosure 12. The blood manifold 22 or 222 is formed to deliver blood to the individual blood receiving channels 58. The manifold 22 or 222 also includes a blood inlet tubing connector 24 to which a suitable tubing, not shown, is coupled for delivering blood to the device 10 from the patient.

The device 10 further includes a gas manifold 26. This gas manifold 26 may either be a separate structure mounted to the device housing enclosure 12, or integrally formed therewith, and is formed to deliver a gas, typically an oxygen-containing gas, i.e. either pure oxygen or blends of oxygen with other suitable gas, e.g. nitrogen, to the gas passage defined by the gas permeable membrane 202.

In accordance with the preferred embodiment as illustrated in Figures 5, 6 and 7a to 7c, hollow fiber membranes 202 are formed in ribbons arranged between adjacently positioned pleats of the bellows 14. These ribbons 208 generally are wrapped about the bellows 14 and the flood manifolds 22 or 222, with the opposing ends of each of the fibers 202 positioned contiguously along the line of the bellows 14 and embedded within a potting polymeric composition, seen at 60. The urethane potting material 60 may be selected from any suitable potting material, e.g., any of the urethane potting materials known in the prior art as in US-A-4715953.

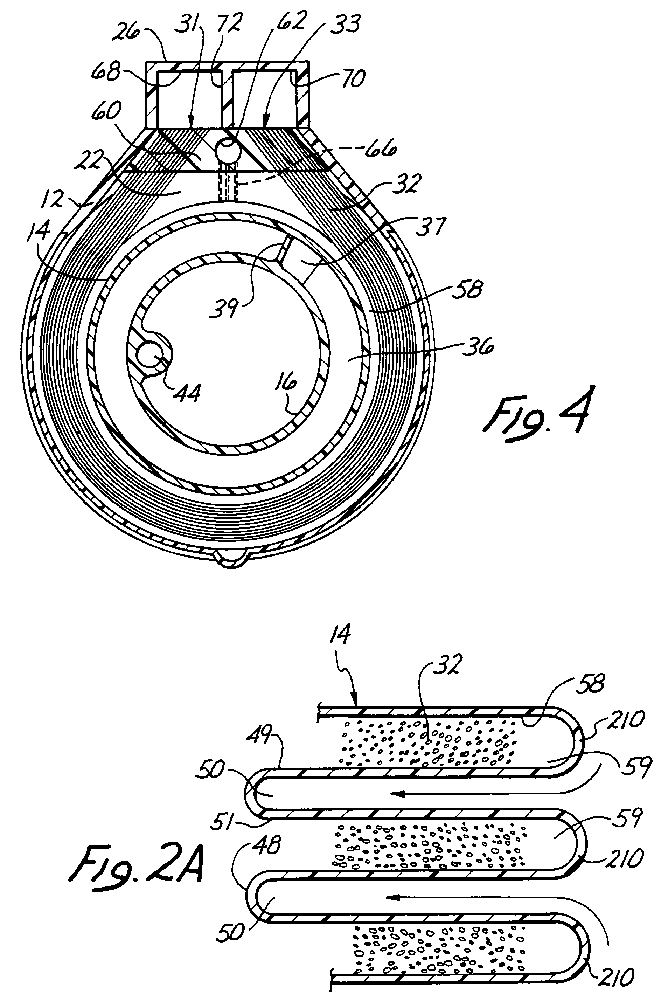

As stated, the opposite open ends of the hollow fibers 202 in ribbons 208 forming hollow fiber bundle 32 are embedded within the potting material in a side by side relationship. This forms rows of the ends of ribbons 208, with such rows seen in Figure 4 at 31 and 33. The respective individual fiber ends are exposed at a surface of the potting material to provide access into the fibers. Typically, after the fibers have been potted a layer of the potting material, in which the fibers are embedded, is removed to expose the open ends of the individual fibers.

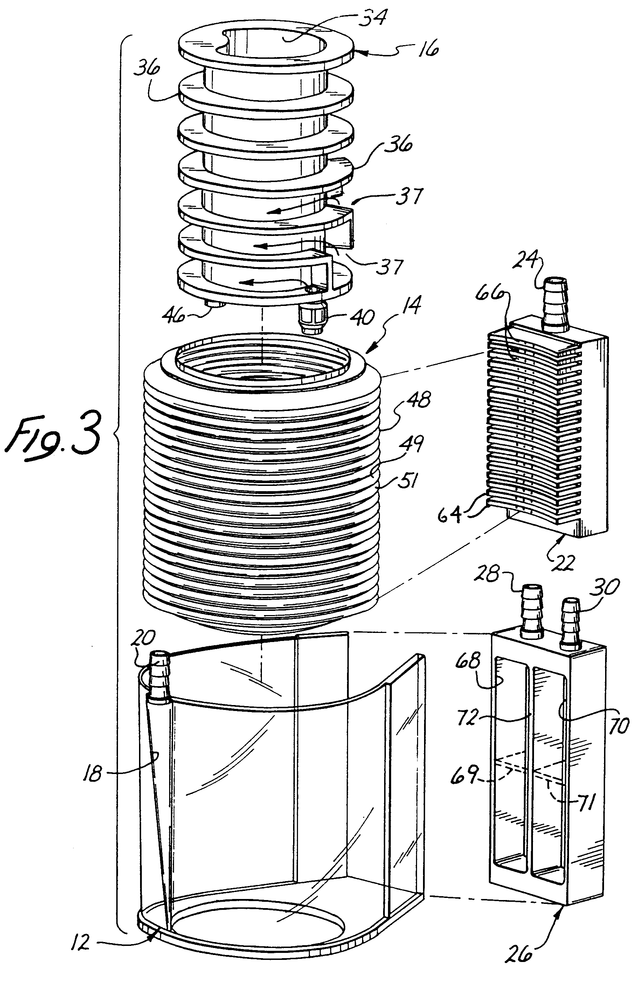

The urethane potting material 60, not only fixes the fibers 202 in position, but also fixes the blood manifold 22 or 222 in the housing 12. One blood manifold 22, is best seen in Figure 3, is an elongated body formed with a centrally disposed conduit 62 running through its length, as seen in Figure 4. This conduit 62 is closed at one end and open at the opposite end to the blood inlet tubing connector 24. The preferred cylindrical blood manifold 222 is shown in Figure 9, also having a conduit 262 and a connector 224.

The blood manifold 22 is formed with a plurality of fingers 64 which extend outward from the same side of the manifold 22. These fingers 64 are spatially separated from each other and have a generally rectangular shape. The fingers 64 are also dimensioned to snugly fit between the adjacently disposed pleats 48 of the bellows 14 between the two side-by-side hollow fiber rows 31 and 33. The preferred blood manifold 222 is in the form of a cylinder adjoining two series of openings 265 along its length, spaced about 120° apart, each pair of openings aligned with a given pleat and having tubing 267 fixed therein and extending into the pleat for blood delivery there. As stated, the manifold is fixed in its position by the urethane potting material 60.

The gas manifold 26 is generally rectangular and formed with two side-by-side hollow compartments, as seen at 68 and 70, partitioned by wall 72. When the manifold 26 is secured to the device 10, each of these compartments 68 and 70 is arranged over rows 31 and 33 respectively of fiber open ends. The manifold 26 includes ports, i.e. 28, to allow a gas, usually oxygen-containing, to be directed into one of these compartments 68 or 70 to then enter the fibers 202 through the open ends, and travel around the bellows 14 through the fibers, and exit through another port, i.e. 30. The compartment 68 and 70, in combination with the individual fibers, define a plurality of separate gas flow pathways around the bellows 14. The gas manifold 26 is mounted to the housing 12 contiguous with the urethane potting material 60.

While the heat exchange fluid pathway 38 is defined by the combination of the jacket 16 or 216 nested in the bellows 14, the blood pathway is defined by the blood receiving channels 58, in combination with the gas exchange members defined by the woven ribbons 208. This is best shown in Figure 7a, and Figure 2A although this shows the hollow fibers arranged in a bundle 32, which is not in accordance with the present invention.

As illustrated in Figure 7a, blood pathway 59 is essentially a gap between ribbons 208 and the base 210 of each pleat. In the preferred embodiment, hollow fiber woven ribbons 208 made as shown in Figures 5 and 6, sized to fit the channel, are laid down in pairs. Ribbons 208A contain 7 hollow fibers 202 and alternate with ribbons 208B, containing 6 hollow fibers 202. The number of fibers, of course, depends on the groove width, but the ribbons are preferably always laid down in alternating layers, with the hollow fibers of each layer for the most part offset with respect to those of adjacent layers to promote enhanced and uniform blood distribution.

Most preferably, 7-fiber ribbon 208A contains first inert fibers 204A which have diameters about one-fifth the diameter of the hollow fiber, while 6-fiber ribbon 208B contains first inert fibers 204B having diameters about one-third the diameter of hollow fibers 202, as shown in Figure 11B to render the hollow fibers of one layer largely offset from those of the adjacent layer. In the embodiment shown in Figure 7B, the ribbons 208 are about 3.18 mm (1/8 inch) wide and the blood channel is about 3.18 mm (1/8 inch) from side 49 to side 51, although of course pleats ranging from about 1.59 mm (1/16 inch) to about 12.7 mm (1/2 inch) or even more can be used. Alternately, layers of identical ribbons 208 having first inert fibers of alternating sizes as shown in Figure 7C, can be used to obtain appropriate spacing.

A spacer 200 in the form of an inert plastic (usually polyester) coil of about 3.18 mm (one-eighth inch) in diameter extends through the pleat in the blood channel 59 adjacent to pleat base 210 for the entire length of the pleat. It maintains the integrity of the blood passage 59 when ribbons 208A and 208B are wound through the pleat during manufacture of the oxygenator and thereafter.

It has been found that sufficient oxygenation is obtained when 32 layers of ribbon 208 are wound in each pleat, the voids about the fibers amounting to about 20 to 80, preferably 40 to 60 percent of the pleat, apart from passageway 59. Preferably 16 layers each of alternating ribbons 208A and 208B are wound in the pleat in pairs to form the 32 alternating, offset layers spaced from the base as shown in Figures 7A, 7B, 7C. While the illustrated embodiment of the blood channel in pleat 58 shown in Figure 7a has a gap 59 between the base 210 and ribbons 208 to define the blood pathway 59, the ribbons 208 may in fact completely fill the individual channels 58. The resulting device 10 would not have blood pathways 59 as seen in the illustrated embodiment. That is, the pathways 59 would not be defined by a gap, but would merely be a pathway formed about the bellows 14 in the blood receiving channels 58 and between the hollow fibers 202. Turning to the blood manifold 22 shown in Figure 4, each finger 64 is formed with one or more open passages 66. Each of these passages 66 communicates with the conduit 62, and with the associated blood pathway 59 when the respective finger 64 is fitted between the pleats 48. Blood manifold 222, preferred, merely provides tubing 267 extending into each pleat. Blood entering the blood manifold 22 or 222 travels through the conduit 62 or 262, and selectively enters and flows through each of the passages 66 or 267. The blood will exit the passages 66 or 267 into the associated blood pathway 59, and travel through the individual blood pathway 59 about the bellows 14. As the gap defining the blood pathway 59 fills, the blood flows outward, about the hollow fibers 202 disposed in ribbons 208 in the associated blood receiving channel 58, and, once oxygenated, exits on the far side through exit 18.

As the blood flows about the individual hollow fibers 202, the exchange of gases occurs. This gas exchange is basically a diffusion process where the oxygen diffuses from the higher concentration in the gas across the fiber membrane to the blood, and the carbon dioxide diffuses from the higher concentration in the blood across the fiber membrane to the gas. The blood will thus become oxygenated as it passes around the individual hollow fibers, and will be subjected to heat exchange by direct contact with the thermally conductive surfaces 49 and 51 of the bellows 14.

The regulation of oxygen and carbon dioxide exchange can be independently controlled by regulating the flow rate of the oxygen-bearing gas through the hollow fibers 202, and by regulating the concentration of the oxygen in such gas. This manner of independently controlling the rate of oxygen and carbon dioxide exchange is known. However, it may be desirable to independently control the rate of carbon dioxide and oxygen exchange in a different manner. Accordingly, in accordance with another embodiment, the gas manifold 26 may be formed with each of the compartments 68 and 70 being further sub-divided into two or more separate portions by partition walls, with such walls being shown in phantom in Figure 3 at 69 and 71. Preferably only that compartment functioning as the gas intake compartment is sub-divided in this manner. That is, only compartment 68 is subdivided into two separate portions. The remaining compartment 70 remains undivided. By introducing an oxygen-bearing gas into only one of the portions of compartment 68, while introducing an inert gas into the other portion, the level of carbon dioxide removal is maintained while the level of oxygen exchange is reduced. This is performed without adjusting the flow rate of the oxygen-bearing gas.

This desire to independently adjust the exchange rates for oxygen and carbon dioxide is particularly advantageous when the patient is under hypothermia, that is when the patient's blood temperature has been lowered. In this state the patient's requirement for oxygen diminishes since the patient's metabolism decreases. Thus the oxygenation requirement diminishes. However, the necessity of removing the carbon dioxide remains constant. The described embodiment of the device of the invention would allow for control in the level of oxygenation by merely fluctuating the delivery of the oxygen-bearing gas and the inert gas to the individual portions of the compartment 68.

It should be noted here that the arrangement of woven ribbons which is described in the preferred embodiment has been found to be extremely effective in oxygen transfer. One of the better high efficiency oxygenators now available on the market contains non-woven microporous fiber believed to be laid down in the winding process described by U.S. patent 4,572,446, and referred to earlier. This winding process produces a fiber pattern in the above-mentioned commercial oxygenator found to have oxygen transfer efficiencies of about 150 ml O₂/minute per square meter of microporous fiber surface area; such an oxygen transfer rate is typical of other high efficiency oxygenators on the market. The woven hollow fiber arrangement described above, however, has been found to produce oxygenators with efficiencies even better than 200 ml O₂/minute per square meter of microporous fiber surface area. Furthermore, in the oxygenator described herein, the priming volume has been reduced to 225 ml of blood as opposed to 325 ml for the above-mentioned commercially available oxygenator.

## Claims

1. Fluid treatment apparatus including a hollow fiber membrane device for the transfer of mass or energy through the device and comprising a housing (12) having first fluid inlet (24) and outlet (18) means and second fluid inlet (28) and outlet means (30), the device comprising hollow fiber membranes (202) extending substantially longitudinally within said housing; said first fluid inlet (24) and outlet (18) means being connected with the hollow fiber membranes (202) for passage of a first fluid therethrough and said second fluid inlet (28) and outlet (30) means being arranged to pass a second fluid over the exterior of the membranes (202) for mass or energy transfer through the membrane, first inert fibers (204) located between said hollow fiber membranes, extending substantially longitudinally and comprising a warp with said hollow fiber membranes; and second inert fibers (206) extending generally transverse to the hollow fiber membranes, generally contiguous therewith and comprising a weft to form a weave with the hollow fiber membranes and first inert fibers;
characterised in that the first inert fibers (204) have a smaller diameter than said hollow fiber membranes, and follow an oscillating path about the second inert fibers (206).

2. Fluid treatment apparatus according to Claim 1 and wherein the first inert fibers (204) are located one between each two hollow fiber membranes so that the warp consists of alternate strands of hollow fiber membrane (202) and first inert fiber (204) and the size and spacing of the first inert fibers define at least in part the amount and flow of the fluid film to be passed over the hollow fiber membranes for mass or energy transfer through said membranes.

3. Fluid treatment apparatus according to Claim 1, wherein each alternate second inert fiber (206) passes over the hollow fiber membranes (202) and the remaining second inert fibers (206) pass under the hollow fiber membranes (202) to form a weft with the first inert fibers passing over and under the second inert fibers in an oscillating pattern.

4. Fluid treatment apparatus according to any preceding claim, wherein each first inert fiber (204) is about one tenth to one third the diameter of the hollow fiber membranes.

5. Fluid treatment apparatus according to Claim 4, wherein each second inert fiber (206) is about one twentieth to one fifth the diameter of the hollow fiber membranes and spaced about five to the inch (2.5 cm).

6. Fluid treatment apparatus according to any preceding claim wherein a plurality of layers (208) of the weave are provided.

7. Fluid treatment apparatus according to Claim 6 wherein the hollow fiber membranes of each layer (208) are generally offset with respect to those of an adjacent layer to promote fluid flow around the hollow fiber membranes.

8. Fluid treatment apparatus according to Claim 6 or 7, wherein first layers (208A) in which the first inert fibers (204A) are about one-fifth the diameter of the hollow fiber membranes (202) alternate with second layers (208B) in which the first inert fibers (204B) are about one-third the diameter of the hollow fiber membranes (202) to promote even and uniform fluid flow around the hollow fiber membranes.

9. Fluid treatment apparatus according to Claim 8 and consisting essentially of multiple layers of said weave, said first layers (208A) each in the form of a ribbon containing a given number of hollow fiber membranes and said second layers (208B) each in the form of a ribbon containing a lesser number of hollow fiber membranes designed so that the hollow fiber membranes are offset from each other in alternate layers.

10. Fluid treatment apparatus according to Claim 7, 8 or 9, wherein the alternating layers contain either six or seven hollow fiber membranes (202) per layer.

11. Fluid treatment apparatus according to Claim 7, 8, 9 or 10 wherein the first inert fibers (204) in each layer vary in size to vary the spacing between hollow fiber membranes (202).

12. Fluid treatment apparatus according to any preceding claim, wherein the hollow fiber membranes (202) are composed of a hydrophobic, biocompatible polymer material permeable to the mass or energy to be transferred through the membrane, but resistant to the flow of liquid through the membrane.

13. Fluid treatment apparatus according to Claim 12, wherein the first inert fibers (204) are composed of a biocompatible monofilament selected to be flexible relative to the hollow fiber, to promote precise spacing of the hollow fibers.

14. Fluid treatment apparatus according to Claim 12 or 13 and wherein the second inert fibers (206) are composed of a monofilament material selected from nylon, polyester, and polypropylene, to promote close controlled spacing between the hollow fibers for largely even, uniform fluid flow over the hollow fiber membranes.

15. Fluid treatment apparatus according to any one of Claims 1 to 11 wherein the hollow fiber membranes (202) are formed of microporous polypropylene and the first and/or second inert fibers (204,206) are formed of polyester.

16. A blood oxygenator comprising a fluid treatment apparatus according to any preceding claim.

17. A blood oxygenator according to Claim 16 wherein the housing (12) includes a channel (58) in which the hollow fiber membrane device is disposed so as to be spaced from a base of the channel to define a gap (59) therein which forms a conduit for passage of blood and from which the blood passes through the membrane device for oxygenation of the blood.

18. A blood oxygenator according to Claim 17, wherein the membrane device in the channel (58) comprises thirty-two woven ribbons (208) having either 6 or 7 of said hollow fiber membranes (202) each, and a coiled spacer (200) extends the length of the channel in the gap (59) to maintain the integrity of the gap (59) and the position of the ribbons during manufacture and use of the oxygenator.

19. A blood oxygenator according to Claim 16 including a heat exchange device (14,16) comprising heat exchange means (14) positioned in said housing (12) for exchanging heat with blood passing through said housing, said exchange means (14) being formed with a plurality of blood flow channels (58), each channel having at least a first thermally conductive wall and including one of said membrane devices.

20. The blood oxygenator of Claim 19, wherein said housing (12) defines an internal chamber and the heat exchange means comprises a thermally conductive core (14) within the chamber, the core (14) being formed with an internal region through which a heat exchange fluid can be directed and with an external surface having a plurality of separate, adjacently positioned, parallel said channels (58), a gas manifold (26) associated with said housing (12) and formed with at least two gas channels (68,70) each communicating with respective ends of the hollow fiber membranes (202) the gas channels communicating with gas inlet and outlet ports (28,30) and a blood manifold (22) mounted in said housing (12) adjacent to said core (14) for delivering blood to said channels (58).

## Patentansprüche

1. Fluidbehandlungsvorrichtung, die eine Hohlfasermembraneinrichtung zum Übertragen von Masse oder Energie durch die Einrichtung hat und ein Gehäuse (12) aufweist, das eine erste Fluideinlaßeinrichtung (24) und Fluidauslaßeinrichtung (18) und eine zweite Fluideinlaßeinrichtung (28) und Fluidauslaßeinrichtung (30) hat, wobei die Einrichtung folgendes aufweist: Hohlfasermembranen (202), die im wesentlichen in Längsrichtung in dem Gehäuse verlaufen; wobei die erste Fluideinlaßeinrichtung (24) und Fluidauslaßeinrichtung (18) mit den Hohlfasermembranen (202) zum Durchfluß eines ersten Fluids durch diese hindurch verbunden sind und die zweite Fluideinlaßeinrichtung (28) und Fluidauslaßeinrichtung (30) angeordnet sind, um ein zweites Fluid über das Äußere der Membranen (202) zu leiten, um Masse oder Energie durch die Membran zu übertragen, erste inerte Fasern (204), die zwischen den Hohlfasermembranen angeordnet sind, im wesentlichen in Längsrichtung verlaufen und eine Kette mit den Hohlfasermembranen aufweisen; und zweite inerte Fasern (206), die im allgemeinen quer zu den Hohlfasermembranen verlaufen, im allgemeinen in Berührung damit sind und einen Schuß aufweisen, um ein Gewebe mit den Hohlfasermembranen und den ersten inerten Fasern zu bilden;
dadurch gekennzeichnet, daß die ersten inerten Fasern (204) einen kleineren Durchmesser als die Hohlfasermembranen haben und einer oszillierenden Bahn um die zweiten inerten Fasern (206) folgen.

2. Fluidbehandlungsvorrichtung nach Anspruch 1, wobei die ersten inerten Fasern (204) so angeordnet sind, daß eine zwischen jeweils zwei Hohlfasermembranen liegt, so daß die Kette aus alternierenden Strängen von Hohlfasermembran (202) und erster inerter Faser (204) besteht und die Größe und Beabstandung der ersten inerten Fasern mindestens teilweise die Menge und den Durchfluß der Fluidschicht definieren, die über die Hohlfasermembranen zu leiten ist, um Masse oder Energie durch die Membranen zu übertragen.

3. Fluidbehandlungsvorrichtung nach Anspruch 1, wobei jede alternierende zweite inerte Faser (206) über die Hohlfasermembranen (202) verläuft und die verbleibenden zweiten inerten Fasern (206) unter den Hohlfasern (202) verlaufen, um einen Schuß mit den ersten inerten Fasern zu bilden, die über und unter den zweiten inerten Fasern in einem oszillierenden Muster verlaufen.

4. Fluidbehandlungsvorrichtung nach einem der vorhergehenden Ansprüche, wobei jede erste inerte Faser (204) einen Durchmesser von ca. 1/10 bis 1/3 des Durchmessers der Hohlfasermembranen hat.

5. Fluidbehandlungsvorrichtung nach Anspruch 4, wobei jede zweite inerte Faser (206) einen Durchmesser von ca. 1/20 bis 1/5 des Durchmessers der Hohlfasermembranen hat und der Abstand derart ist, daß ungefähr fünf pro 2,5 cm (1 inch) vorhanden sind.

6. Fluidbehandlungsvorrichtung nach einem der vorhergehenden Ansprüche, wobei eine Vielzahl von Lagen (208) des Gewebes vorgesehen ist.

7. Fluidbehandlungsvorrichtung nach Anspruch 6, wobei die Hohlfasermembranen jeder Lage (208) im allgemeinen in bezug auf diejenigen einer angrenzenden Lage versetzt sind, um den Fluiddurchfluß um die Hohlfasermembranen herum zu fördern.

8. Fluidbehandlungsvorrichtung nach Anspruch 6 oder 7, wobei erste Lagen (208A), in denen die ersten inerten Fasern (204A) einen Durchmesser von ca. 1/5 des Durchmessers der Hohlfasermembranen (202) haben, mit zweiten Lagen (208B) alternieren, in denen die ersten inerten Fasern (204B) einen Durchmesser von ca. 1/3 des Durchmessers der Hohlfasermembranen (202) haben, um einen gleichmäßigen und gleichförmigen Fluiddurchfluß um die Hohlfasermembranen herum zu fördern.

9. Fluidbehandlungsvorrichtung nach Anspruch 8, die im wesentlichen aus einer Vielzahl von Lagen des Gewebes besteht, wobei die ersten Lagen (208A), die jeweils in Form eines Bands sind, eine gegebene Anzahl von Hohlfasermembranen enthalten und die zweiten Lagen (208B), die jeweils in Form eines Bands sind, eine geringere Anzahl von Hohlfasermembranen enthalten und so ausgebildet sind, daß die Hohlfasermembranen in alternierenden Lagen zueinander versetzt sind.

10. Fluidbehandlungsvorrichtung nach Anspruch 7, 8 oder 9, wobei die alternierenden Lagen entweder sechs oder sieben Hohlfasermembranen (202) pro Lage enthalten.

11. Fluidbehandlungsvorrichtung nach Anspruch 7, 8, 9 oder 10, wobei die ersten inerten Fasern (204) in jeder Lage in ihrer Größe verschieden sind, damit der Abstand zwischen Hohlfasermembranen (202) variiert.

12. Fluidbehandlungsvorrichtung nach einem der vorhergehenden Ansprüche, wobei die Hohlfasermembranen (202) aus einem hydrophoben, biokompatiblen Polymermaterial bestehen, das für die durch die Membran zu übertragende Masse oder Energie durchlässig ist, dem Durchfluß von Flüssigkeit durch die Membran jedoch Widerstand bietet.

13. Fluidbehandlungsvorrichtung nach Anspruch 12, wobei die ersten inerten Fasern (204) aus einem biokompatiblen Monofilament bestehen, das ausgewählt ist, um relativ zu der Hohlfaser flexibel zu sein, um eine präzise Beabstandung der Hohlfasern zu fördern.

14. Fluidbehandlungsvorrichtung nach Anspruch 12 oder 13, wobei die zweiten inerten Fasern (206) aus einem Monofilamentmaterial bestehen, das aus Nylon, Polyester und Polypropylen ausgewählt ist, um eine enge kontrollierte Beabstandung zwischen den Hohlfasern für weitgehend gleichmäßigen, gleichförmigen Fluiddurchfluß über die Hohlfasermembranen zu fördern.

15. Fluidbehandlungsvorrichtung nach einem der Ansprüche 1 bis 11, wobei die Hohlfasermembranen (202) aus mikroporösem Polypropylen gebildet sind und die ersten und/oder zweiten inerten Fasern (204, 206) aus Polyester gebildet sind.

16. Blutoxygenator, der eine Fluidbehandlungsvorrichtung nach einem der vorhergehenden Ansprüche aufweist.

17. Blutoxygenator nach Anspruch 16, wobei das Gehäuse (12) einen Kanal (58) aufweist, in dem die Hohlfasermembraneinrichtung so angeordnet ist, daß sie von einer Basis des Kanals beabstandet ist, um einen Zwischenraum (59) darin zu definieren, der eine Leitung für den Durchfluß von Blut bildet und aus dem das Blut durch die Membraneinrichtung zur Oxygenierung des Bluts fließt.

18. Blutoxygenator nach Anspruch 17, wobei die Membraneinrichtung in dem Kanal (58) zweiunddreißig gewebte Bänder (208) aufweist, die jeweils entweder sechs oder sieben der Hohlfasermembranen (202) haben, und ein gewickelter Abstandshalter (200) entlang der Länge des Kanals in dem Zwischenraum (59) verläuft, um die Integrität des Zwischenraums (59) und die Position der Bänder während der Herstellung und des Gebrauchs des Oxygenators aufrechtzuerhalten.

19. Blutoxygenator nach Anspruch 16, der eine Wärmeaustauscheinrichtung (14, 16) aufweist, die einen in dem Gehäuse (12) positionierten Wärmetauscher (14) zum Austausch von Wärme mit durch das Gehäuse fließendem Blut aufweist, wobei der Wärmetauscher (14) mit einer Vielzahl von Blutdurchflußkanälen (58) ausgebildet ist, wobei jeder Kanal mindestens eine erste wärmeleitfähige Wand hat und eine der Membraneinrichtungen aufweist.

20. Blutoxygenator nach Anspruch 19, wobei das Gehäuse (12) eine innere Kammer definiert und der Wärmetauscher folgendes aufweist: einen wärmeleitfähigen Kern (14) in der Kammer, wobei der Kern (14) mit einem inneren Bereich, durch den ein Wärmeaustauschfluid geleitet werden kann, und mit einer äußeren Oberfläche ausgebildet ist, die eine Vielzahl von getrennten, einander benachbart positionierten, parallelen Kanälen (58) hat, eine Gassammelleitung (26), die dem Gehäuse (12) zugeordnet und mit mindestens zwei Gaskanälen (68, 70) ausgebildet ist, die jeweils mit entsprechenden Enden der Hohlfasermembranen (202) in Verbindung stehen, wobei die Gaskanäle mit Gaseinlaß- und Gasauslaßöffnungen (28, 30) in Verbindung stehen, und eine Blutsammelleitung (22), die in dem Gehäuse (12) angrenzend an den Kern (14) angebracht ist, um den Kanälen (58) Blut zuzuführen.

## Revendications

1. Appareil de traitement des fluides incluant un dispositif de membrane à fibres creuses pour le transfert de masse ou d'énergie à travers le dispositif et comprenant une enveloppe (12) comportant un premier organe d'entrée (24) et de sortie (18) de fluide et un second organe d'entrée (28) et de sortie (30) de fluide, le dispositif comprenant des membranes à fibres creuses (202) s'étendant sensiblement longitudinalement dans ladite enveloppe; ledit premier organe d'entrée (24) et de sortie (18) de fluide étant relié aux membranes à fibres creuses (202) pour permettre le passage d'un premier fluide et ledit second organe d'entrée (28) et de sortie (30) de fluide étant disposé pour faire passer un second fluide sur l'extérieur des membranes (202) pour le transfert de masse ou d'énergie à travers la membrane, de premières fibres inertes (204) situées entre lesdites membranes à fibres creuses, s'étendant sensiblement longitudinalement et comprenant une chaîne avec lesdites membranes à fibres creuses; et de secondes fibres inertes (206) s'étendant généralement transversalement aux membranes à fibres creuses, généralement contiguës avec elles et comprenant une trame pour former une armure avec les membranes à fibres creuses et les premières fibres inertes;
caractérisé en ce que les premières fibres inertes (204) ont un plus faible diamètre que les dites membranes a fibres creuses, et suivent un trajet oscillant autour des secondes fibres inertes (206).

2. Appareil de traitement des fluides selon la revendication 1 et dans lequel les premières fibres inertes (204) sont situées chacune entre deux membranes à fibres creuses de manière que la chaîne consiste en brins alternés de membrane à fibres creuses (202) et de première fibre inerte (204) et que la taille et l'espacement des premières fibres inertes définissent au moins en partie la quantité et le débit de film de fluide à faire passer sur les membranes à fibres creuses pour le transfert de masse ou d'énergie à travers lesdites membranes.

3. Appareil de traitement des fluides selon la revendication 1, dans lequel une fibre inerte (206) sur deux passe sur les membranes a fibres creuses (202) et les secondes fibres inertes (206) restantes passent sous les membranes à fibres creuses (202) pour former une trame avec les premières fibres inertes passant sur et sous les secondes libres inertes dans un schéma oscillant.

4. Appareil de traitement des fluides selon l'une quelconque des revendications précédentes dans lequel chaque première fibre inerte (204) a un diamètre d'environ un dixième à un tiers du diamètre des membranes à fibres creuses.

5. Appareil de traitement des fluides selon la revendication 4, dans lequel chaque seconde fibre inerte (206) a un diamètre d'environ un vingtième à un cinquième du diamètre des membranes à fibres creuses et est espacée à raison d'environ cinq pour 2,5 cm.

6. Appareil de traitement des fluides selon l'une quelconque des revendications précédentes dans lequel sont fournies plusieurs couches (208) d'armure.

7. Appareil de traitement des fluides selon la revendication 6 dans lequel les membranes à fibres creuses de chaque couche (208) sont généralement décalées par rapport à celles d'une couche adjacente pour favoriser l'écoulement de fluide autour des membranes à fibres creuses.

8. Appareil de traitement des fluides selon la revendication 6 ou 7, dans lequel de premières couches (208A) dans lesquelles les premières fibres inertes (204A) ont un diamètre d'environ un cinquième du diamètre des membranes à fibres creuses (202) alternent avec de secondes couches (208B) dans lesquelles les premières fibres inertes (204B) ont un diamètre d'environ un tiers du diamètre des membranes à fibres creuses (202) pour favoriser un écoulement de fluide égal et uniforme autour des membranes à fibres creuses.

9. Appareil de traitement des fluides selon la revendication 8 et consistant essentiellement en couches multiples de ladite armure, lesdites premières couches (208A) étant chacune sous la forme d'un ruban contenant un nombre donné de membranes à fibres creuses et lesdites secondes couches (208B) étant chacune sous la forme d'un ruban contenant un nombre plus faible de membranes à fibres creuses conçues de manière que les membranes à fibres creuses soient décalées l'une par rapport à l'autre en couches alternées.

10. Appareil de traitement des fluides selon la revendication 7, 8 ou 9, dans lequel les couches alternant contiennent six ou sept membranes à fibres creuses (202) par couche.

11. Appareil de traitement des fluides selon la revendication 7, 8, 9 ou 10 dans lequel les premières fibres inertes (204) dans chaque couche varient en taille pour faire varier l'espacement entre les membranes à fibres creuses (202).

12. Appareil de traitement des fluides selon l'une quelconque des revendications précédentes, dans lequel les membranes à fibres creuses (202) sont composées d'une matière polymérique hydrophobe, biocompatible perméable à la masse ou à l'énergie à transférer à travers la membrane, mais résistant à l'écoulement de liquide à travers la membrane.

13. Appareil de traitement des fluides selon la revendication 12, dans lequel les premières fibres inertes (204) sont composées d'un monofilament biocompatible choisi pour être flexible relativement à la fibre creuse, pour favoriser un espacement précis des fibres creuses.

14. Appareil de traitement des fluides selon la revendication 12 ou 13 et dans lequel les secondes fibres inertes (206) sont composées d'une matière monofilamentaire choisie parmi le nylon, le polyester, et le polypropylène, pour favoriser un espacement étroitement contrôlé entre les fibres creuses pour un écoulement de fluide largement égal et uniforme sur les membranes à fibres creuses.

15. Appareil de traitement des fluides selon l'une quelconque des revendications 1 à 11 dans lequel les membranes à fibres creuses (202) sont formées de polypropylène microporeux et les premières et/ou secondes libres inertes (204, 206) sont formées de polyester.

16. Oxygénateur de sang comprenant un appareil de traitement des fluides selon l'une quelconque des revendications précédentes.

17. Oxygénateur de sang selon la revendication 16 dans lequel l'enveloppe (12) inclut un canal (58) dans lequel le dispositif de membrane à fibres creuses est disposé de manière à être espacé de la base du canal pour y définir un intervalle (59) qui forme un conduit pour le passage du sang et à partir duquel le sang passe à travers le dispositif à membrane pour l'oxygénation du sang.

18. Oxygénateur de sang selon la revendication 17, dans lequel le dispositif à membrane dans le canal (58) comprend trente-deux rubans tissés (208) ayant chacun 6 ou 7 desdites membranes à fibres creuses (202), et une entretoise enroulée (200) s'étend le long du canal dans l'intervalle (59) pour maintenir l'intégrité de l'intervalle (59) et la position des rubans au cours de la fabrication et de l'utilisation de l'oxygénateur.

19. Oxygénateur de sang selon la revendication 16 incluant un dispositif d'échange de chaleur (14, 16) comprenant un organe d'échange de chaleur (14) positionné dans ladite enveloppe (12) pour échanger de la chaleur lorsque le sang passe à travers ladite enveloppe, ledit organe d'échange (14) étant formé avec plusieurs canaux d'écoulement de sang (58), chaque canal ayant au moins une première paroi conductrice de la chaleur et incluant l'un desdits dispositifs à membrane.

20. Oxygénateur de sang de la revendication 19, dans lequel ladite enveloppe (12) définit une chambre interne et l'organe d'échange de chaleur comprend un noyau conducteur de la chaleur (14) dans la chambre, le noyau (14) étant formé avec une région interne à travers laquelle un fluide d'échange thermique peut être dirigé et avec une surface externe ayant plusieurs desdits canaux (58) séparés parallèles, positionnés de manière adjacente, un collecteur de gaz (26) associé à ladite enveloppe (12) et formé avec au moins deux canaux de gaz (68, 70) communiquant chacun avec les extrémités respectives des membranes à fibres creuses (202), les canaux de gaz communiquant avec des ouvertures d'entrée et de sortie de gaz (28, 30) et un collecteur de sang (22) monté dans ladite enveloppe (12) au voisinage dudit noyau (14) pour fournir du sang auxdits canaux (58).
